# EUROPÄISCHE PATENTSCHRIFT

(11) **EP 1 165 231 B1**
(45) Veröffentlichungstag und Bekanntmachung des Hinweises auf die Patenterteilung: **22.10.2003**
(21) Anmeldenummer: 00909274.3
(22) Anmeldetag: 01.03.2000
(51) Int. Cl.: B01J 23/755, B01J 37/03, B01J 33/00

(54) **NICKEL-KATALYSATOR ZUR HYDRIERUNG FUNKTIONELLER GRUPPEN UND VERFAHREN ZU SEINER HERSTELLUNG**
NICKEL CATALYST FOR HYDROGENATING FUNCTIONAL GROUPS AND METHOD FOR PRODUCING SAME
CATALYSEUR AU NICKEL DESTINE A L'HYDROGENATION DE GROUPES FONCTIONNELS ET SON PROCEDE DE PRODUCTION

(30) Priorität: 03.03.1999 DE 19909177
(43) Veröffentlichungstag der Anmeldung: 02.01.2002
(73) Patentinhaber: KataLeuna GmbH Catalysts, 06236 Leuna (DE)
(72) Erfinder: BIRKE, Peter, D-06179 Langenbogen (DE); GEYER, Reinhard, D-06120 Halle (DE); KRAAK, Peter, D-04159 Leipzig (DE); SCHÖDEL, Rainer, D-06179 Teutschenthal (DE)
(74) Vertreter: Schrell, Andreas, Dr.
(86) Internationale Anmeldenummer: EP0001708
(87) Internationale Veröffentlichungsnummer: WO00051727

(56) Entgegenhaltungen:
- EP-A- 0 597 662
- US-A- 3 868 332
- US-A- 3 988 262
- US-A- 4 090 980
- US-A- 4 634 515
- US-A- 4 668 654
- US-A- 4 956 328

## Beschreibung

Die Erfindung betrifft einen Katalysator, der für die Hydrierung von funktionellen Gruppen organischer Verbindungen in Gegenwart von Wasser, insbesondere für die Hydrierung von Nitrogruppen in Nitroaromaten zu den entsprechenden Aminen in Gegenwart von Wasser oder für die Hydrierung von Aldosen und Ketosen zu den entsprechenden Zuckeralkoholen in Gegenwart von Wasser, eingesetzt werden kann, und ein Verfahren zu dessen Herstellung.

Die technisch häufigsten Anwendungen der Hydrierung funktioneller Gruppen organischer Verbindungen stellen die Hydrierung von Aldosen oder Ketosen zu den entsprechenden Zuckeralkoholen beziehungsweise von Nitroaromaten zu den entsprechenden Aminen dar.

Die Hydrierungen werden im allgemeinen sowohl im Festbettreaktor als auch im Batch-Reaktor durchgeführt. In technischem Maßstab werden am häufigsten Hydrierungen in der Flüssigphase mit suspendiertem Katalysator vorgenommen, wobei die Verfahren sich durch die Reaktionstemperatur, den Druck, den Katalysator, die Lösungsmittel und die Art der Reaktionsführung unterscheiden. Als Katalysatoren finden dabei verschiedene Katalysatorsysteme Anwendung, wie beispielsweise Nickel-haltige Katalysatoren. Die katalytische Hydrierung von Glucose zu Sorbitol mit einem Nickel-SiO₂-Al₂O₃-Katalysator wird in der NL 8 102 190 offenbart. Der Einsatz von Nickel-Kupfer-Trägerkatalysatoren für die Glucosehydrierung ist in der DD 217 996 offenbart. Als Träger werden SiO₂, Al₂O₃ und SiO₂ · Al₂O₃ verwendet. In der DD 156 175 wird die Sorbitolgewinnung durch Hydrierung von Glucose in Gegenwart eines Ni-SiO₂-Katalysators beschrieben. Entsprechend der Patentschrift SU 565 040 verläuft die Hydrierung zu Sorbitol an Raney-Nickel-Katalysatoren bei einer Katalysatorkonzentration von 5 bis 6 %, Temperaturen von 110 bis 150°C und Drücken von 40 bis 60 bar nach 1 bis 2 Stunden unter Erhalt guter Ausbeuten. Die US 4,694,113 beschreibt einen Zweistufenprozeß für die Hydrierung von Glucose zu Sorbitol, wobei in der ersten Stufe etwa 95 % der Glucose in Gegenwart eines Nickel-Katalysators zu Sorbitol hydriert werden und nach Abtrennung des Nickel-Katalysators der Rest der Glucose mit einem Ru-Katalysator zu Sorbitol hydriert wird.

Die JP 551 33 33 offenbart die Hydrierung von 2,4-Dinitrotoluol und 2,6-Dinitrotoluol in Gegenwart der Katalysatoren Pd/C, Raney-Nickel, Raney-Kobalt beziehungsweise Platinschwarz.

Die EP-A 98 681 offenbart einen Nickel-Kieselgur-Trägerkatalysator für die Hydrierung von Di-Nitrobenzophenon zum entsprechenden Diamin.

In der DE-A 35 37 247 wird die Hydrierung von Di-Nitroverbindungen zu den Diaminen in Gegenwart von modifizierten Raney-Nickel-Katalysatoren beschrieben.

Die EP-A 0 335 222 offenbart die Anwendung von Nickel-Al₂O₃/ZrO₂-Trägerkatalysatoren für die Hydrierung von Nitrilen, Aromaten, Nitroverbindungen und Olefinen. Die Schrift offenbart unter anderem die gleichzeitige Fällung von Nickel, Zirconium und Aluminium auf Trägern bei 50 bis 120°C und bei einem pH-Wert von 7,3 bis 9,0, wobei als Träger Aktivkohle, Al₂O₃, SiO₂, Kieselgur und andere eingesetzt werden.

Die SU-PS 28 31 85 offenbart Nickel-Al₂O₃/ZrO₂-Katalysatoren, die durch Fällen von Nickel und Al₂O₃ auf ZrO₂ hergestellt wurden.

Gemäß der Lehre der US-PS 2,564,331 wird ein Nickel-ZrO₂-Katalysator durch Fällen eines Nickelund Zirconylcarbonatgemisches mit anschließendem Waschen, Trocknen und Reduzieren bei 250 bis 350°C hergestellt, wobei der Katalysator maximal 10 Masse-% ZrO₂ aufweist.

Auch in der DE-AS 1 257 753 wird die Fällung unlöslicher Carbonate offenbart, wobei der Fällvorgang durch Verdampfen von CO₂ und NH₃ aus einer Mischsalzlösung von Ammoniumzirconylcarbonat und Nickelammincarbonat ausgelöst wird.

Die EP-A 0 672 452 offenbart Katalysatoren zur Hydrierung organischer Verbindungen, die im wesentlichen 65 bis 80 Masse-% Nickel, berechnet als NiO, 10 bis 25 Masse-% SiO₂, 2 bis 10 Masse-% Zirconium, berechnet als ZrO₂ und 0 bis 10 Masse-% Aluminium, berechnet als Al₂O₃, enthalten, wobei die Summe aus dem Gehalt an SiO₂ und Al₂O₃ mindestens 15 Masse-% beträgt. Die Herstellung dieser Katalysatoren erfolgt durch Zugabe einer sauren wäßrigen Lösung von Ni-, Zr- und gewünschtenfalls Aluminiumverbindungen zu einer basischen wäßrigen Lösung oder Suspension von Siliciumverbindungen und gewünschtenfalls Aluminiumverbindungen. Während der Fällung wird der pH-Wert zunächst auf 4,0 bis 6,5 abgesenkt und nachfolgend auf 7 bis 8 eingestellt. Das Fällprodukt wird getrocknet, calciniert und verformt.

Die bisher bekannten Nickel-Hydrierkatalysatoren weisen alle den Nachteil auf, daß unter den hydrothermalen Reaktionsbedingungen sowohl der Glucoseals auch der Nitroaromatenhydrierung eine schnelle Alterung der Katalysatoren auftritt.

Das der vorliegenden Erfindung zugrunde liegende technische Problem liegt also darin, Nickel-haltige Trägerkatalysatoren bereitzustellen, die insbesondere unter den hydrothermalen Reaktionsbedingungen der Glucose- und Nitroaromatenhydrierung eine höhere Lebensdauer als die herkömmlichen Katalysatoren aufweisen.

Dieses Problem wird erfindungsgemäß dadurch gelöst, daß ein Katalysator, insbesondere für die Hydrierung von funktionellen Gruppen von organischen Verbindungen, insbesondere für die Hydrierung von Glucose zu Sorbitol oder von Nitrogruppen in Nitroaromaten zu den entsprechenden Aminen in Gegenwart von Wasser, enthaltend Nickel auf einem Träger bestehend aus ZrO₂, ZrO₂HfO₂, SiO₂ · ZrO₂, SiO₂ · ZrO₂HfO₂ oder Gemischen mindestens zweier Substanzen davon bereitgestellt wird, wobei der Katalysator reduziert und stabilisiert ist, Nickelkristallite mit monomodaler Nickelkristallitgrößenverteilung, einen Nickelgehalt von 25 bis 60 Masse-% (bezogen auf die Gesamtmasse des Katalysators), insbesondere 25 bis 59 Masse-% (bezogen auf die Gesamtmasse des Katalysators) einen ZrO₂-Gehalt 20 bis 75 Masse-% (bezogen auf die Gesamtmasse des Katalysators), sowie einen Reduktionsgrad von mindestens 65 % aufweist. Der Reduktionsgrad wird nach einer einstündigen Nachreduktion des stabilisierten Katalysators bei 100°C im Wasserstoffstrom (Belastung: 1000 v/vh) bestimmt.

Die Erfindung löst dieses Problem auch durch die Bereitstellung eines Verfahrens zur Herstellung eines solchen Katalysators.

Die Erfindung sieht in einer besonders bevorzugten Ausführungsform vor, daß der vorgenannte Katalysator eine monomodale Nickelkristallitgrößenverteilung aufweist, wobei das Maximum der Nickelkristallitgrößenverteilung bei 25 bis 90 Angström, insbesondere 30 bis 90 Angström liegt.

In einer besonders bevorzugten Ausführungsform beträgt der SiO₂-Gehalt 0 bis 40 Masse-% (bezogen auf die Gesamtmasse des Katalysators). In einer weiteren bevorzugten Ausführungsform beträgt der HfO₂-Gehalt 0 bis 7,5 Masse-% (bezogen auf die Gesamtmasse des Katalysators).

In einer besonders bevorzugten Ausführungsform der Erfindung können die reduzierten und stabilisierten Katalysatoren als Pulver mit Korngrößen von 1 bis 100 um, vorzugsweise von 2 bis 30 µm, eingesetzt werden. Selbstverständlich können auch Formlinge eingesetzt werden.

Die erfindungsgemäßen Katalysatoren zeichnen sich in vorteilhafter und überraschender Weise durch ihre gegenüber herkömmlichen Katalysatoren verlängerte Lebensdauer bei gleicher oder verbesserter katalytischer Aktivität aus. Katalysatoren der erfindungsgemäßen monomodalen Nickelkristallitgrößenverteilung weisen insbesondere unter hydrothermalen Reaktionsbedingungen eine erheblich verlängerte Lebensdauer gegenüber herkömmlichen Katalysatoren auf.

Im Zusammenhang mit der vorliegenden Erfindung wird unter einer monomodalen Nickelkristallitgrößenverteilung eine Verteilung der Nickelkristallite verstanden, gemäß der lediglich ein Maximum der Kristallitgrößenverteilung vorliegt.

Im Zusammenhang mit der vorliegenden Erfindung wird unter dem Begriff Reduktionsgrad der Anteil des metallischen Nickels am Gesamtnickelgehalt des stabilisierten Katalysators verstanden, der nach einstündiger Nachreduktion bei 100°C vorliegt.

Die Erfindung betrifft in einer weiteren Ausführungsform auch ein Verfahren zur Herstellung des vorgenannten Katalysators. Die Erfindung betrifft also auch ein Verfahren zur Herstellung eines Nickel-haltigen Trägerkatalysatores, insbesondere eines Katalysators für die Hydrierung von Carbonylgruppen in Aldosen oder Ketosen in Gegenwart von Wasser und von Nitrogruppen in Nitroaromaten zu den entsprechenden Aminen in Gegenwart von Wasser, wobei durch Fällen aus einer Ni²⁺- und Zr⁴⁺-haltigen Lösung mit einer basischen Lösung, insbesondere einer Lösung von NaOH, NaHCO₃ oder Na₂CO₃ oder eines Gemisches mindestens zweier dieser Substanzen, bis zu einem pH-Wert von 8 bis 9 ein Fällprodukt erhalten wird, welches bei Temperaturen von 300°C bis 650°C calciniert, gegebenenfalls anschließend inertisiert, und nachfolgend mit Wasserstoff bei Temperaturen von 250°C bis 550°C, insbesondere 300°C bis 550°C, reduziert, gegebenenfalls inertisiert, und anschließend stabilisiert wird.

In einer besonders bevorzugten Ausführungsform enthält die Ni²⁺- und Zr⁴⁺-haltige Lösung zusätzlich Hf⁴⁺. In einer weiteren bevorzugten Ausführungsform enthält die Ni²⁺ und Zr²⁺-haltige Lösung oder die Ni²⁺ und Zr⁴⁺/Hf⁴⁺-haltige Lösung Siliciumdioxid SiO₂, vorzugsweise in suspendierter Form. In bevorzugter Ausführungsform kann vorgesehen sein, daß die Ni²⁺- und Zr⁴⁺-haltige Lösung Nitrate aufweist, insbesondere in Form von Zirconylnitrat.

Die Herstellung des Fällprodukts erfolgt also durch Zugabe der genannten basischen Lösung zu der Ni²⁺und Zr⁴⁺-haltigen Lösung, wobei diese Zugabe soweit und solange erfolgt, bis die Mischung der beiden Lösungen einen pH-Endwert von 8 bis 9 erreicht.

Die Erfindung sieht in einer bevorzugten Ausführungsform vor, daß die Fällung bei Temperaturen von 60°C bis 95°C erfolgt. In bevorzugter Ausführung kann vorgesehen sein, nach der durchgeführten Fällung, also dem Erreichen des pH-Endwertes, die erhaltene Suspension zum Beispiel für 1 bis 2 Stunden nachzurühren, bevor eine Weiterbearbeitung erfolgt.

In einer weiteren Ausbildung betrifft die Erfindung ein vorgenanntes Verfahren, wobei das Fällprodukt nach der Fällung filtriert, gewaschen, vorzugsweise mit Wasser, und nachfolgend bei Temperaturen von 110°C bis 150°C in nicht-reduzierender Atmosphäre getrocknet und ein Vorläuferkatalysator erhalten wird.

Im Zusammenhang mit der vorliegenden Erfindung wird unter einem Vorläuferkatalysator ein Produkt verstanden, das nach der Fällung der Ausgangskomponenten, also der Ni²⁺- und Zr⁴⁺-haltigen, gegebenenfalls Hf⁴⁺-haltigen Lösung, und gegebenenfalls des SiO₂ mit der zugesetzten basischen Lösung, Filtration, Waschen mit Wasser und Trocknung bei Temperaturen in nicht-reduzierender Atmosphäre erhalten wird.

Erfindungsgemäß ergeben sich bei der Herstellung des Vorläuferkatalysators Phasen aus Nickelhydroxynitrat (Ni₃(OH)₄(NO₃)₂) oder Nickelhydroxynitrat (Ni₃(OH)₄(NO₃)₂-haltige Phasen, insbesondere Gemische aus Nickelhydroxynitrat (Ni₃(OH)₄(NO₃)₂), Nickelhydroxycarbonat (Ni₂(OH)₂CO₃ 4H₂O) und Nickelhydroxysilikat (Ni₃Si₂O₅(OH)₄) oder Gemische aus Nickelhydroxynitrat (Ni₃(OH)₄(NO₃)₂) und Nickelhydroxid (Ni(OH)₂) mit Gitteraufweitungen oder eine Sepiolith-ähnliche Struktur (Ni₄Zr₆O₁₅(OH)₂), wobei OH⁻-Ionen partiell durch Carbonationen ausgetauscht sein können.

Im Zusammenhang mit der vorliegenden Erfindung wird unter Gitteraufweitung eine Interferenzlagenverschiebung zu kleineren Winkeln verstanden.

Entweder vor oder nach der Calcination kann der Katalysatorvorläufer zu Tabletten, Strängen, Kissen, Kugeln oder ähnlichem verformt werden.

Die Reduktion des calcinierten Produktes kann erfindungsgemäß sowohl am Pulver als auch an den Formlingen erfolgen. Erfindungsgemäß ist es besonders bevorzugt, während der Reduktion Gasbelastungen im Bereich von 500 bis 3000 v/v h einzusetzen.

Erfindungsgemäß ist in bevorzugter Ausführung vorgesehen, daß die Katalysatoren nach der Reduktion, vorzugsweise mit einem O₂-N₂-CO₂-Gemisch, stabilisiert werden.

Die Erfindung betrifft daher auch die Bereitstellung eines Verfahrens zur Passivierung eines, vorzugsweise reduzierten und/oder vorzugsweise inertisierten erfindungsgemäßen Katalysators, wobei der Katalysator in einem Verfahrensschritt a) in einem CO₂-N₂-Gasgemisch mit einem CO₂-Gehalt von 0,5 bis 10 Vol.-% bei Temperaturen von 91°C bis 350°C mindestens 30 Minuten behandelt, in einem Verfahrensschritt b) anschließend in dem in Schritt a) genannten Gasgemisch auf eine Temperatur von maximal 90°C abgekühlt, anschließend in einem Verfahrensschritt c) nach Erreichen der Temperatur von maximal 90°C in einer ersten Passivierungsphase dem Gasgemisch Sauerstoff, vorzugsweise Luft, bis zu einem Gehalt an 0,2 bis 1,5 Vol.-% Sauerstoff zugesetzt und der Katalysator in dem Gemisch mindestens 30 Minuten unter Rütteln behandelt und anschließend in einem Verfahrensschritt d) der CO₂-Gehalt in dem Gasgemisch gemäß Schritt c) in einer zweiten Passivierungsphase auf <0,1 Vol.-% reduziert und der O₂-Gehalt auf 1,5 bis 21 Vol.-% erhöht wird.

Die erfindungsgemäße Verfahrensweise zur Stabilisierung des Katalysators weist den Vorteil kurzer Stabilisierungszeiten auf, wobei gleichzeitig gut reaktivierbare Katalysatoren mit sehr guter thermischer Stabilität erhalten werden. In vorteilhafter Weise werden die Katalysatoren besonders gleichmäßig passiviert. Tatsächlich war es überraschend, daß durch das Behandeln mit CO₂-armen Inertgasen unter den angegebenen Bedingungen sehr gleichmäßig und leicht reaktivierbare Katalysatoren erhalten wurden.

Die Erfindung betrifft in einer bevorzugten Ausführungsform ein vorgenanntes Verfahren, wobei zumindest die Passivierung in einem Katalysatorbett kontinuierlich oder im Batch-Verfahren durchgeführt wird, insbesondere mit einem Katalysatorbett, dessen Höhe zum Durchmesser-Verhältnis im Bereich von 0,05 bis 1 liegt.

In einer weiteren bevorzugten Ausführungsform sieht die Erfindung ein vorgenanntes Verfahren vor, wobei die Konzentration des CO₂ während der Behandlung mit dem CO₂-N₂-Gemisch gemäß des Verfahrensschrittes a) 1 bis 2,5 Vol.-% beträgt.

In einer weiteren bevorzugten Ausführungsform sieht die Erfindung ein vorgenanntes Verfahren vor, wobei die Gasbelastung während der Behandlung mit dem CO₂-N₂-Gemisch gemäß des Verfahrensschrittes a) 500 bis 10000 v/v h beträgt. In einer weiteren bevorzugten Ausführungsform sieht die Erfindung vor, daß das vorgenannte Verfahren eine Gasbelastung während der Behandlung mit dem CO₂-N₂-Gemischs gemäß des Verfahrensschritts a) und/oder während der Behandlung mit dem CO₂-N₂-O₂-Gasgemisch gemäß der Verfahrensschritte c) und d) 100 bis 3000 v/v h beträgt.

Die Erfindung sieht in einer weiteren bevorzugten Ausführungsform vor, daß das vorgenannte Verfahren die Behandlung in dem CO₂-N₂-O₂-Gasgemisch gemäß der Verfahrensschritte c) und d) für einen Zeitraum von 30 Minuten bis 8 Stunden durchgeführt wird.

Die Erfindung betrifft in einer weiteren Ausbildung ein vorgenanntes Verfahren, wobei die zeitliche Dauer der Behandlung gemäß des Verfahrensschrittes c), also der ersten Passivierungsphase, zu der zeitlichen Dauer des Verfahrensschrittes gemäß des Verfahrensschrittes d), also der zweiten Passivierungsphase, 9:1 beträgt.

In einer weiteren bevorzugten Ausführungsform betrifft die Erfindung ein vorgenanntes Verfahren, wobei die Temperatur der Behandlung des Katalysators mit dem CO₂-N₂-O₂-Gasgemisch gemäß Schritt c) und/oder Schritt d) 50 bis 70°C beträgt.

In einer weiteren bevorzugten Ausführungsform sieht die Erfindung ein vorgenanntes Verfahren vor, wobei die CO₂-Konzentration im CO₂-N₂-O₂-Gasgemisch während der Behandlung gemäß des Verfahrensschrittes c) 0,5 bis 1,5 Vol.-% beträgt. Die Erfindung kann in bevorzugter Weise vorsehen, den CO₂-Gehalt des Gemisches aus Schritt a) für die Durchführung des Schrittes c) herabzusetzen, zum Beispiel auf den vorgenannten Bereich.

Gemäß einer weiteren bevorzugten Ausbildung der vorliegenden Erfindung wird ein vorgenanntes Verfahren bereitgestellt, wobei die O₂-Konzentration im CO₂-N₂-O₂-Gasgemisch während der Behandlung gemäß des Verfahrensschrittes c) 0,25 bis 0,8 Vol.-% beträgt.

In einer weiteren bevorzugten Ausbildung der Erfindung beträgt die O₂-Konzentration während der Behandlung gemäß des Verfahrensschrittes d) 5 bis 10 Vol.-%.

Die Erfindung betrifft in einer weiteren Ausgestaltung ein vorgenanntes Verfahren, wobei vorgesehen ist, daß das Rütteln des Katalysatorbettes gemäß der Verfahrensschritte c) und/oder d) in Zeitabständen von 10 bis 20 Minuten über einen Zeitraum von jeweils 0,5 bis 2 Minuten vorgenommen wird. Es ist vorteilhaft, Rüttelfrequenzen von 10 bis 50 Hz einzustellen.

Selbstverständlich ist es auch möglich, insbesondere bei pulverförmigen Katalysatoren und Katalysatoren mit sehr hohen Festigkeiten, das Katalysatorbett durch Erzeugen einer Wirbelschicht oder durch Anordnung in einem Drehrohrofen in Bewegung zu setzen. Ein wesentlicher Gesichtspunkt der vorliegenden Erfindung ist es, den Katalysator zumindest zeitweise während der Passivierungsphasen gemäß der Verfahrensschritte c) und d) in dem Sauerstoff-Kohlenstoffdioxid-Stickstoff-Gemisch zu bewegen, beispielsweise in einem Bewegtbett.

Die Stabilisierung kann in einer besonders bevorzugten Weise auch durchgeführt werden, indem in einem Stickstoffstrom mit einem Sauerstoffgehalt von 0,1 bis 1 Vol.-% und einem CO₂-Gehalt von 0,6 Vol.-% bei Temperaturen unterhalb von 80°C stabilisiert wird.

Selbstverständlich ist es möglich, die Stabilisierung des erfindungsgemäß erhaltenen reduzierten Katalysators auch in anderer Weise durchzuführen, beispielsweise gemäß der Lehre der US-PS 4,090,980, die hinsichtlich der Verfahrensparameter zur Stabilisierung von Katalysatoren in den Offenbarungsgehalt der vorliegenden Anmeldung mit einbezogen ist.

Die Erfindung wird anhand der folgenden Beispiele näher erläutert:

### Beispiel 1 (erfindungsgemäß)

4,5 l Wasser werden in einen mit einem Rührer versehenen, beheizbaren Fällbehälter vorgelegt und anschließend eine Natriumbicarbonatlösung, die durch Lösen von 1,5 kg NaHCO₃ in 10 1 Wasser hergestellt wurde, und eine Wasserglaslösung (45 g SiO₂ = 0,75 l Lösung) zugegeben. Danach wird unter Rühren auf eine Fälltemperatur von 85°C aufgeheizt und mit der Dosierung einer vereinigten Metallnitratlösung, die neben 400 g Nickel auch 158 g ZrO₂ und 27 g HfO₂ in Form der Nitrate enthielt, begonnen. Die Fällzeit bei einer durchschnittlichen Temperatur von 80 - 85°C betrug ca. 2 h. Der pH-Wert nach der Fällung lag bei 8,2 - 8,5. Nach Abschluß der Fällung wurde die fertige Suspension noch ca. 2 h bei 80°C nachgerührt. Im Anschluß daran wird die Suspension filtriert und der Filterkuchen bis zu einem Na₂O - Gehalt von < 0,3 Masse-%, bezogen auf den Glührückstand des bei 800°C getemperten Produkts, mit alkalifreiem Wasser gewaschen. Danach wird der Filterkuchen ca. 14 h bei Temperaturen von 120 bis 150°C getrocknet und nachfolgend 2 h bei 370°C calciniert. Nach der Calcinierung wird das Zwischenprodukt im Stickstoffstrom (1000 v/v h) inertisiert, im Wasserstoffstrom (1000 v/v h) mit einer Aufheizrate von 5°C/min auf 460°C hochgeheizt und 6h bei 460°C reduziert. Anschließend wurde 30 Minuten bei 460°C im Stickstoffstrom mit 1500 v/v h inertisiert, danach im Stickstoffstrom (1500 v/v h) auf 290°C abgekühlt, bei dieser Temperatur wird dem Stickstoff Kohlendioxid in einer solchen Menge zugesetzt, daß die CO₂-Konzentration 2 Vol-% beträgt.

Der Katalysator wird mit diesem Gemisch 30 Minuten bei 280°C behandelt, nachfolgend im gleichen Gasgemisch auf 50°C abgekühlt und in einem Stickstoffstrom (1500 v/v h) mit einem Sauerstoffgehalt von 0,1 bis 1 Vol-% und einem CO₂-Gehalt von 0,6 Vol-% bei Temperaturen unterhalb 80°C stabilisiert. Die Sauerstoffkonzentration wurde so gewählt, daß die Katalysatortemperatur 80°C nicht überstieg. Die Stabilisierungszeit bei Temperaturen < 80°C betrug 5h.

Der reduzierte und stabilisierte Katalysator enthält ca. 50 Masse-% Nickel, 20 Masse-% ZrO₂, 5 Masse-% SiO₂ und 3 Masse-% HfO₂. Die XRD-Untersuchungen ergaben eine monomodale Nickelkristallitgrößenverteilung mit einem Maximum von 42 Angström. Der Reduktionsgrad des Katalysators beträgt nach einer einstündigen Nachreduktion bei 100°C 78 % (Belastung im Wasserstoffstrom: 1000 v/v h). Die Phasenanalyse des getrockneten Filterkuchens ergab das Vorliegen der sepiolithähnlichen Struktur : Ni₄Zr₆O₁₅(OH)₂ (mit partiellem Ersatz der OH⁻-Ionen durch Carbonationen).

### Beispiel 2 (erfindungsgemäß)

4,5 l Wasser werden in den beheizbaren Fällbehälter vorgelegt und anschließend eine Metallnitratlösung, die neben 400 g Nickel auch Zirconium und Hafnium in Form einer Nitratlösung enthält, zugegeben. Das Molverhältnis von Nickel zu ZrO₂ in der Metallnitratlösung beträgt ca. 2, das Molverhältnis ZrO₂ zu HfO₂ ca. 25. Nach der Zugabe der Metallnitratlösung wird unter Rühren auf eine Temperatur von 60°C aufgeheizt und anschließend mit einer wäßrigen Sodalösung (150 g Soda/l Lösung) bis zum End-pH-Wert von ca. 8,5 gefällt. Die Fällzeit betrug ebenfalls ca. 2 h. Nach Abschluß der Fällung wurde bei Temperaturen von 60 - 70°C nachgerührt, anschließend filtriert und weiter, wie unter Beispiel 1 beschrieben, bearbeitet.

Der fertige Katalysator enthält ca. 40 Masse-% Nickel, 40 Masse-% ZrO₂ und 3 Masse-% HfO₂. Die monomodale Ni-Kristallitgrößenverteilung besitzt ein Maximum bei 38 Angström. Der Reduktionsgrad des Katalysators beträgt nach einer einstündigen Nachreduktion bei 100°C 80 %. Die Phasenanalyse des getrockneten Filterkuchens ergab ein Gemisch aus Nickelhydroxynitrat (Ni₃(OH)₄(NO₃)₂) und Nickelhydroxycarbonat (Ni₂(OH)₂CO₃4H₂O)

### Beispiel 3 (Vergleichsbeispiel)

305,76 g Ni (NO₃) 6H₂O und 29,52 g Al (NO₃)₃ 9H₂O werden in 1760 ml destilliertem Wasser und 2,32 g Zirconcarbonat in 9 ml HNO₃ (56 Masse-%) gelöst. Beide Lösungen werden vereinigt und auf 101°C erhitzt. Diese Mischsalzlösung wird innerhalb von 3 Minuten gleichmäßig zu einer 100°C heißen und intensiv gerührten Sodalösung gegeben, die aus 147,04 g Na₂CO₃ und 1416 ml destilliertem Wasser hergestellt worden ist. In die frischgefällte Suspension werden 27,76 g Kieselgur eingerührt und die dabei entstehende Mischung wird noch weitere 3 Minuten gerührt. Das Fällprodukt wird anschließend filtriert und mit 70°C heißem Wasser gewaschen, bis der Alkaligehalt des Waschwassers ca. 20 mg Na₂O/l beträgt. Der auf diese Weise erhaltene Filterkuchen wird in 70°C heißem Wasser suspendiert (Mengenverhältnis Filterkuchen zu Wasser = 1:1), 60 Minuten gerührt und danach erneut filtriert. Der danach anfallende Filterkuchen wird erneut filtriert. Der hierbei entstehende Filterkuchen wird zu zylinderförmigen Formkörpern (Durchmesser 5 mm, Länge 8 bis 10 mm) extrudiert und anschließend bei steigender Temperatur (50 bis 60°C) mit Luft auf einen Restgehalt an Wasser < 10 Masse-%, bezogen auf getrocknete Masse, getrocknet. Das getrocknete Material wird im Wasserstoffstrom nach vorheriger Inertisierung im Stickstoffstrom (1000 v/v h, 30 min) mit einer Belastung von 400 v/v h und einer Aufheizzeit von 5°C/min auf 470°C hochgeheizt und über einen Zeitraum von 4 h bei dieser Temperatur reduziert. Im getrockneten Zwischenprodukt wurden die Phasen Takovit (Carbonatvariante) und Nickelhydroxysilikat mit Carbonateinbau nachgewiesen. Die Stabilisierung wurde wie in den erfindungsgemäßen Beispielen vorgenommen. Die Eigenschaften des Katalysators sind in der Tabelle denen der erfindungsgemäßen Katalysatoren gegenübergestellt.

### Beispiel 4 (Vergleichsbeispiel)

2,7 kg Soda wurden in 6 l Wasser aufgeschlämmt und auf 60°C erwärmt. Innerhalb von 30 Minuten wurden unter starkem Rühren 6,6 l einer Lösung von Natriumsilikat mit einem Gehalt von 45 g SiO₂/l und anschließend 3 l einer Nickelnitratlösung mit einem Gehalt von 150 g Ni/l Lösung innerhalb von 2 h zugegeben. Die Fällsuspension wurde dann 1 h bei 60°C nachgerührt. Danach wurden weitere 3,5 l einer Nickelnitratlösung mit einem Gehalt von 150 g Ni/l Lösung innerhalb von 2 h in die Fällsuspension eingeleitet. Der Niederschlag wurde abfiltriert und mit Wasser gewaschen. Anschließend wurde der Filterkuchen 10 h bei 130°C getrocknet, im Wasserstoffstrom nach vorheriger Calcinierung und Inertisierung, wie im Beispiel 1 beschrieben, 6 h bei 450°C reduziert, nach Inertisierung im Stickstoffstrom (1000 v/v h, 30 min bei 450°C) und nach Abkühlen im Stickstoffstrom unter Einsatz eines 0,1 - 1 % O₂ enthaltenden Stickstoffstroms (1500 v/v h) bei Temperaturen < 80°C stabilisiert. Die Stabilisierungzeit betrug 8 h. Der reduzierte und stabilisierte Katalysator enthält ca. 62 % Nickel und 20 % SiO₂. Der Reduktionsgrad des Katalysators beträgt nach einer einstündigen Nachreduktion bei 100°C 75 %.

Die Phasenanalyse des getrockneten Filterkuchens ergab im wesentlichen Nickelhydroxysilikat mit Carbonateinbau.

Die katalytische Charakterisierung der Katalysatoren wurde unter folgenden Bedingungen vorgenommen:
A) Hydrierung von Glucose im 0,5 l Rührautoklav mit Wasserstoffverbrauchsmessung bei konstantem Druck:

| | |
|---|---|
| Katalysatormenge | 1,2 g |
| Reaktionsgemisch | 120 g Glucose und 90 g Wasser |
| Reaktionsdruck | 125 bar |
| Reaktionstemperatur | 135°C |
| Rührgeschwindigkeit | 2000 Umdrehungen/Minute |

Als Maß für die Hydrieraktivität diente die Zeit, in der 98,5 % der Glucose hydriert worden sind.
B)Hydrierung von Nitrobenzol zu Anilin im 0,5 l Autoklav mit Wasserstoffverbrauchsmessung bei konstantem Druck:

| | |
|---|---|
| Katalysatormenge | 0,25 g |
| Reaktionsgemisch | 80 g, 40 g Wasser |
| Reaktionsdruck | 25 bar |
| Reaktionstemperatur | 120°C |
| Rührgeschwindigkeit | 2000 Umdrehungen / Minute |

Als Maß für die Hydrieraktivität diente die Zeit, in der 100 % des Nitrobenzols umgesetzt worden sind.

Die Stabilität der Katalysatoren wurde durch die Zunahme der durchschnittlichen Ni-Kristallitgröße charakterisiert, die sich nach einer 100-stündigen Behandlung im Reaktionsgemisch nach der Hydrierreaktion unter den Druck- und Temperaturbedingungen der Hydrierung einstellt.

Die XDR-Weitwinkel-Untersuchungen zur qualitativen Phasenzuordnung wurden unter den folgenden experimentellen Aufnahmebedingungen an einem Meßplatz der Fa. Rich. Seifert & CO Freiberger Präzisionsmechanik GmbH ausgeführt:

| | |
|---|---|
| Generatordaten | 34kV/30mA |
| Goniometer | HZG4 |
| Strahlung | Cu-Kₐ |
| Filter | gebogener Graphitmonochromator |
| Winkelbereich | 2 Θ = 10° - 70° |
| Schrittweite | Δ Θ = 0,05° |
| Zählzeit | 4s |

Die Bearbeitung der Daten wurde im Auswertefile APX63 (SEIFERT FPM) durchgeführt. Zur Zuordnung der kristallinen Strukturen wurde der JCPDS-Auswertefile 1997 herangezogen.

Ebenfalls mit einem Meßplatz der Fa. Rich. Seifert & CO Freiberger-Präzisionsmechanik GmbH wurde die mittlere Primärteilchengröße des Nickels bestimmt, wobei die Streukurvenausschnitte senkrecht zur (111)-Netzebene aus der Interferenzlinienverbreitung unter folgenden Bedingungen aufgenommen worden sind:

| | |
|---|---|
| Generatordaten | 40kV/30mA |
| Goniometer | XDR7 |
| Strahlung | Cu-Kₐ |
| Filter | Ni |
| Winkelbereich | 2 Θ = 41° - 49° |
| Schrittweite | Δ Θ = 0,05° |
| Zählzeit | 20s |

Durch Anwendung des Peakentflechtungsprogrammes PF4 der Firma Jandel Corporation wurden Aussagen zur Modalität (Monogauslinienprofil oder bimodales Gauslinienprofil) des Ni-(111)-Linienprofils gewonnen.

Die Charakterisierung der Katalysatoren ergibt sich aus folgender Tabelle:

| Katalysator | Hydrierzeit f. Nitrobenzol in min (Umsatz: 100 %) | Anilinausbeute in % | Hydrierzeit f. Glucose in min (Umsatz: 98,5 %) | mittlere Ni-Kristallitgröße in Angström (vor der Reaktion) | mittlere Ni-Kristallitgröße in Angström (nach dem Stabilitäts test) |
|---|---|---|---|---|---|
| Beispiel 1 | 97 | 99,2 | 41 | 48 | 61 |
| Beispiel 2 | 95 | 99,1 | 43 | 56 | 65 |
| Beispiel 3 Vergleichsbeispiel | 129 | 98,6 | 52 | 107 | 134 |
| Beispiel 4 Vergleichsbeispiel | 115 | 98,7 | 50 | 72 | 107 |

Die Ergebnisse zeigen die Vorzüge der erfindungsgemäßen Katalysatoren, die in ihrer hohen katalytischen Aktivität und hohen Stabilität liegen, wie die geringe Zunahme der Ni-Kristallitgrößen belegt.

## Patentansprüche

1. Katalysator, insbesondere für die Hydrierung von funktionellen Gruppen von organischen Verbindungen in Gegenwart von Wasser, enthaltend Nickel auf einem Träger bestehend aus ZrO₂, ZrO₂HfO₂, SiO₂ · ZrO₂ SiO₂ · ZrO₂HfO₂ oder Gemischen zweier oder mehrerer Substanzen davon, wobei der Katalysator stabilisiert ist, Nickelkristallite mit monomodaler Nickelkristallitgrößenverteilung, einen Nickelgehalt von 25 bis 60 Masse-% (bezogen auf die Gesamtmasse des Katalysators), einen ZrO₂-Gehalt von 20 bis 75 Masse-% (bezogen auf die Gesamtmasse des Katalysators) sowie einen Reduktionsgrad von mindestens 65% aufweist.

2. Katalysator nach Anspruch 1, wobei das Maximum der Nickelkristallitgrößenverteilung bei 25 bis 90 Angström liegt.

3. Katalysator nach einem der vorhergehenden Ansprüche, wobei der SiO₂-Gehalt maximal 40 Masse-% (bezogen auf die Gesamtmasse des Katalysators) beträgt.

4. Katalysator nach einem der vorhergehenden Ansprüche, wobei der HfO₂-Gehalt maximal 7,5 Masse-% (bezogen auf die Gesamtmasse des Katalysators) beträgt.

5. Katalysator nach einem der vorhergehenden Ansprüche, wobei der Katalysator in Form eines Pulvers, insbesondere mit Korngrößen von 1 bis 100 um, vorzugsweise 2 bis 30 µm vorliegt.

6. Verfahren zur Herstellung eines Nickel-haltigen Trägerkatalysators nach einem der Ansprüche 1 bis 5, insbesondere für die Hydrierung von funktionellen Gruppen von organischen Verbindungen in Gegenwart von Wasser, enthaltend Nickel auf einem Zirconium-haltigen Träger wie im Anspruch 1 definiert, wobei der Katalysator stabilisiert ist, Nickel-Kristallite mit monomodaler Nickelkristallitgrößenverteilung, einen Nickelgehalt von 25 bis 60 Masse-% (bezogen auf die Gesamtmasse des Katalysators), einen ZrO₂-Gehalt von 20 bis 75 Masse-% (bezogen auf die Gesamtmasse des Katalysators) sowie einen Reduktionsgrad von mindestens 65 % aufweist, wobei durch Fällen aus einer Ni²⁺- und Zr⁴⁺-haltigen Lösung mit einer basischen Lösung bis zu einem pH-Endwert von 8 bis 9 ein Fällprodukt erhalten wird, welches bei Temperaturen von 300°C bis 650°C calciniert, mit Wasserstoff bei Temperaturen von 250°C bis 550°C reduziert und stabilisiert wird.

7. Verfahren nach Anspruch 6, wobei das Fällprodukt nach dem Calcinieren und vor dem Reduzieren inertisiert wird.

8. Verfahren nach einem der Ansprüche 6 bis 7, wobei die basische Lösung eine Lösung von NaOH, NaH-CO₃, Na₂CO₃ oder eines Gemisches zweier oder mehrerer dieser Substanzen ist.

9. Verfahren nach einem der Ansprüche 6 bis 8, wobei die Ni²⁺- und Zr⁴⁺-haltige Lösung NO₃⁻ (Nitrat) und/oder Hf⁴⁺ enthält.

10. Verfahren nach einem der Ansprüche 6 bis 9, wobei die Ni²⁺- und Zr⁴⁺-haltige Lösung SiO₂, vorzugsweise in suspendierter Form, enthält.

11. Verfahren nach einem der Ansprüche 6 bis 10, wobei die Fällung bei Temperaturen von 60°C bis 95°C erfolgt.

12. Verfahren nach einem der Ansprüche 6 bis 11, wobei das Fällprodukt nach der Fällung filtriert, gewaschen, vorzugsweise mit Wasser, nachfolgend in nicht-reduzierender Atmosphäre getrocknet, vorzugsweise bei Temperaturen von 110°C bis 150°C, und ein Vorläuferkatalysator erhalten wird.

13. Verfahren nach einem der Ansprüche 6 bis 12, wobei bei der Herstellung des Vorläuferkatalysators Phasen aus Nickelhydroxynitrat (Ni₃(OH)₄(NO₃)₂) oder Nickelhydroxynitrat- (Ni₃(OH)₄(NO₃)₂) haltige Phasen, insbesondere Gemische aus Nickelhydroxynitrat (Ni₃(OH)₄(NO₃)₂), Nickelhydroxycarbonat (Ni₂(OH)₂CO₃ 4H₂O) und Nickelhydroxysilikat (Ni₃Si₂O₅(OH)₄) oder Gemische aus Nickelhydroxynitrat (Ni₃(OH)₄(NO₃)₂) und Nickelhydroxid (Ni(OH)₂) mit Gitteraufweitungen oder eine Sepiolith-ähnliche Struktur (Ni₄Zr₆O₁₅(OH)₂), wobei OH⁻-Ionen partiell durch Carbonationen ausgetauscht sein können, ausgebildet werden.

14. Verfahren nach einem der Ansprüche 6 bis 13, wobei der Katalysatorvorläufer vor der Calcination zu Tabletten, Strängen, Kissen oder Kugeln geformt wird.

15. Verfahren nach einem der Ansprüche 6 bis 14, wobei der Katalysatorvorläufer nach der Calcination zu Tabletten, Strängen, Kissen oder Kugeln geformt wird.

16. Verfahren nach einem der Ansprüche 6 bis 15, wobei die Gasbelastung bei der Reduktion 500 bis 3000 v/v h beträgt.

17. Verfahren nach einem der Ansprüche 6 bis 16, wobei die Katalysatoren mit einem O₂-N₂-CO₂-Gemisch stabilisiert werden, insbesondere mit einem O₂-N₂-CO₂-Gemisch mit einem Sauerstoffgehalt von 0,1 bis 1 Vol.-% und einem CO₂-Gehalt von 0,6 Vol.-%.

18. Verfahren nach einem der Ansprüche 6 bis 17, wobei die Stabilisierung bei Temperaturen unterhalb 80°C stattfindet.

## Claims

1. Catalyst, especially for hydrogenating functional groups of organic compounds in the presence of water, containing nickel on a support consisting of ZrO₂, ZrO₂HfO₂, SiO₂•ZrO₂, SiO₂•ZrO₂HfO₂ or mixtures of two or more of these substances, wherein the catalyst is stabilised and provides nickel crystallites with monomodal nickel-crystallite size distribution, a nickel content of 25 to 60% by mass (relative to the total mass of the catalyst), a ZrO₂ content from 20 to 75% by mass (relative to the total mass of the catalyst) and a degree of reduction of at least 65%.

2. Catalyst according to claim 1, wherein the maximum of the nickel-crystallite size distribution is at 25 to 90 Ångström.

3. Catalyst according to any one of the preceding claims, wherein the maximum SiO₂-content is 40% by mass (relative to the total mass of the catalyst).

4. Catalyst according to any one of the preceding claims, wherein the maximum HfO₂-content is 7.5% by mass (relative to the total mass of the catalyst).

5. Catalyst according to any one of the preceding claims, wherein the catalyst is present in the form of a powder, especially with grain sizes from 1 to 100µm, preferably 2 to 30µm.

6. Method for producing a nickel-containing, supported catalyst according to any one of claims 1 to 5, especially for hydrogenating functional groups of organic compounds in the presence of water, containing nickel on a zirconium- containing support as defined in claim 1, wherein the catalyst is stabilised and provides nickel crystallites with monomodal nickel-crystallite size distribution, a nickel content of 25 to 60% by mass (relative to the total mass of the catalyst), a ZrO₂-content of 20 to 75% by mass (relative to the total mass of the catalyst) and a degree of reduction of at least 65%, wherein a precipitation product is obtained by precipitation from a solution containing Ni²⁺ and Zr⁴⁺ with a basic solution up to a final pH-value from 8 to 9, which calcines at temperatures from 300°C to 650°C and is reduced and stabilised with hydrogen at temperatures from 250°C to 550°C.

7. Method according to claim 6, wherein the precipitation product is rendered inert after calcination and before reduction.

8. Method according to any one of claims 6 to 7, wherein the basic solution is a solution of NaOH, NaHCO₃, Na₂CO₃ or a mixture of two or more of these substances.

9. Method according to any one of claims 6 to 8, wherein the Ni²⁺- and Zr⁴⁺-containing solution contains NO₃⁻ (nitrate) and/or Hf⁴⁺.

10. Method according to any one of claims 6 to 9, wherein the Ni²⁺- and Zr⁴⁺-containing solution contains SiO₂, preferably in suspended form.

11. Method according to any one of claims 6 to 10, wherein the precipitation takes place at temperatures from 60°C to 95°C.

12. Method according to any one of claims 6 to 11, wherein, after precipitation, the precipitation product is filtered, washed, preferably with water and subsequently dried in a non-reducing atmosphere, preferably at temperatures from 110°C to 150°C, and a precursor catalyst is obtained.

13. Method according to any one of claims 6 to 12, wherein, during the manufacture of the precursor catalyst, phases consisting of nickel hydroxy nitrate (Ni₃(OH)₄(NO₃)₂) or phases containing nickel hydroxy nitrate (Ni₃(OH)₄(NO₃)₂), especially mixtures of nickel hydroxy nitrate (Ni₃(OH)₄(NO₃)₂), nickel hydroxy carbonate (Ni₂(OH)₂CO₃4H₂O) and nickel hydroxy silicate (Ni₃Si₂O₅(OH)₄) or mixtures of nickel hydroxy nitrate (Ni₃(OH)₄(NO₃)₂) and nickel hydroxide (Ni(OH)₂) with lattice expansions, or a structure similar to sepiolite (Ni₄Zr₆O₁₅(OH)₂), wherein OH⁻ ions can be partially substituted by carbonate ions, are formed.

14. Method according to any one of claims 6 to 13, wherein the catalyst precursor is formed into tablets, strands, lumps or balls before calcination.

15. Method according to any one of claims 6 to 14, wherein the catalyst precursor is formed into tablets, strands, lumps or balls after calcination.

16. Method according to any one of claims 6 to 15, wherein the gas loading during reduction amounts to 500 to 3000 v/v h.

17. Method according to any one of claims 6 to 16, wherein the catalysts are stabilised with an O₂-N₂-CO₂-mixture, especially with an O₂-N₂-CO₂-mixture with an oxygen content from 0.1 to 1% by volume and a CO₂ content of 0.6% by volume.

18. Method according to any one of claims 6 to 17, wherein the stabilisation takes place at temperatures below 80°C.

## Revendications

1. Catalyseur, en particulier pour l'hydrogénation de groupes fonctionnels de composés organiques en présence d'eau, contenant du nickel sur un support constitué de ZrO₂, ZrO₂HfO₂, SiO₂ . ZrO₂, SiO₂ . ZrO₂HfO₂, ou de mélanges de deux substances ou plus parmi celles-ci, dans lequel le catalyseur est stabilisé, présente des cristallites de nickel à distribution monomodale de la taille des cristallites de nickel, une teneur en nickel de 25 à 60 % en masse (par rapport à la masse totale du catalyseur), une teneur en ZrO₂ de 20 à 75 % en masse (par rapport à la masse totale du catalyseur), ainsi qu'un degré de réduction d'au moins 65 %.

2. Catalyseur selon la revendication 1, dans lequel le maximum de la distribution de la taille des cristallites de nickel se situe de 25 à 90 Angströms.

3. Catalyseur selon l'une quelconque des revendications précédentes, dans lequel le teneur en SiO₂ est au maximum de 40 % en masse (par rapport à la masse totale du catalyseur).

4. Catalyseur selon l'une quelconque des revendications précédentes, dans lequel la teneur en HfO₂ est au maximum de 7,5 % en masse (par rapport à la masse totale du catalyseur).

5. Catalyseur selon l'une quelconque des revendications précédentes, dans lequel le catalyseur est sous forme de poudre, en particulier d'une granulométrie de 1 à 100 µm, de préférence de 2 à 30 µm.

6. Procédé de préparation d'un catalyseur sur support contenant du nickel selon l'une quelconque des revendications 1 à 5, en particulier pour l'hydrogénation de groupes fonctionnels de composés organiques en présence d'eau, contenant du nickel sur un support contenant du zirconium tel que défini dans la revendication 1, dans lequel le catalyseur est stabilisé, présente des cristallites de nickel à distribution monomodale de la taille des cristallites de nickel, une teneur en nickel de 25 à 60 % en masse (par rapport à la masse totale du catalyseur), une teneur en ZrO₂ de 20 à 75 % en masse (par rapport à la masse totale du catalyseur), ainsi qu'un degré de réduction d'au moins 65 %, dans lequel on obtient, par précipitation à partir d'une solution contenant du Ni²⁺ et du Zr⁴⁺ avec une solution basique jusqu'à une valeur finale de pH de 8 à 9, un produit de précipitation qui est calciné à des températures de 300 à 650°C, réduit par de l'hydrogène à des températures de 250 à 550°C et stabilisé.

7. Procédé selon la revendication 6, dans lequel le produit de précipitation est inertisé après la calcination et avant la réduction.

8. Procédé selon l'une quelconque des revendications 6 à 7, dans lequel la solution basique est une solution de NaOH, de NaHCO₃, de Na₂CO₃ ou un mélange de deux de ces substances ou plus.

9. Procédé selon l'une quelconque des revendications 6 à 8, dans lequel la solution contenant du Ni²⁺ et du Zr⁴⁺ contient du NO₃⁻ (nitrate) et/ou du Hf⁴⁺.

10. Procédé selon l'une quelconque des revendications 6 à 9, dans lequel la solution contenant du Ni²⁺ et du Zr⁴⁺ contient du SiO₂, de préférence sous forme en suspension.

11. Procédé selon l'une quelconque des revendications 6 à 10, dans lequel la précipitation a lieu à des températures de 60°C à 95°C.

12. Procédé selon l'une quelconque des revendications 6 à 11, dans lequel le produit de précipitation est, après la précipitation, filtré, lavé, de préférence à l'eau, séché ensuite dans une atmosphère non réductrice, de préférence à des températures de 110 à 150°C, et l'on obtient un précurseur de catalyseur.

13. Procédé selon l'une quelconque des revendications 6 à 12, dans lequel se forment, lors de la préparation du précurseur de catalyseur, des phases constituées d'hydroxynitrate de nickel (Ni₃(OH)₄(NO₃)₂) ou des phases contenant de l'hydroxynitrate de nickel (Ni₃(OH)₄(NO₃)₂), en particulier des mélanges d'hydroxynitrate de nickel (Ni₃(OH)₄(NO₃)₂), d'hydroxycarbonate de nickel (Ni₂(OH)₂CO₃4H₂O) et d'hydroxysilicate de nickel (Ni₃Si₂O₅(OH)₄) ou des mélanges d'hydroxynitrate de nickel (Ni₃(OH)₄(NO₃)₂) et d'hydroxyde de nickel (Ni(OH)₂) avec des élargissements du réseau ou une structure similaire à celle de la sépiolite (Ni₄Zr₆O₁₅(OH)₂), dans lequel des ions OH⁻ peuvent être partiellement échangés par des ions carbonate.

14. Procédé selon l'une quelconque des revendications 6 à 13, dans lequel le précurseur de catalyseur est, avant la calcination, façonné en pastilles, en granulés cylindriques, en coussinets ou en billes.

15. Procédé selon l'une quelconque des revendications 6 à 14, dans lequel le précurseur de catalyseur est, après la calcination, façonné en pastilles, en granulés cylindriques, en coussinets ou en billes.

16. Procédé selon l'une quelconque des revendications 6 à 15, dans lequel la charge de gaz lors de la réduction est de 500 à 3000 v/v h.

17. Procédé selon l'une quelconque des revendications 6 à 16, dans lequel les catalyseurs sont stabilisés par un mélange de O₂-N₂-CO₂, en particulier par un mélange de O₂-N₂-CO₂ présentant une teneur en oxygène de 0,1 à 1 % en volume et une teneur en CO₂ de 0,6 % en volume.

18. Procédé selon l'une quelconque des revendications 6 à 17, dans lequel la stabilisation a lieu à des températures inférieures à 80°C.
